# EUROPÄISCHE PATENTSCHRIFT

(11) **EP 1 137 341 B1**
(45) Veröffentlichungstag und Bekanntmachung des Hinweises auf die Patenterteilung: **25.02.2004**
(21) Anmeldenummer: 99963404.1
(22) Anmeldetag: 07.12.1999
(51) Int. Cl.: A23C 9/152, A23L 1/304, A61P 37/04, A23L 1/305, A61K 33/30, A23J 1/00

(54) **THYMUSWACHSTUMSSTIMULIERENDE SÄUGLINGSNAHRUNG**
BABY FOOD STIMULATING GROWTH OF THE THYMUS
ALIMENT POUR NOURRISSON STIMULANT LA CROISSANCE DU THYMUS

(30) Priorität: 09.12.1998 DE 19856789
(43) Veröffentlichungstag der Anmeldung: 04.10.2001
(73) Patentinhaber: N.V. Nutricia, 2712 HM Zoetermeer (NL)
(72) Erfinder: SAWATZKI, Günther, D-35516 Münzenberg (DE); BÖHM, Günther, D-61209 Echzell (DE); GEORGI, Gilda, D-61381 Friedrichsdorf (DE)
(74) Vertreter: Köster, Hajo, Dr.
(86) Internationale Anmeldenummer: PCT/EP1999/009565
(87) Internationale Veröffentlichungsnummer: WO 2000/033662

(56) Entgegenhaltungen:
- EP-A- 0 048 473
- EP-A- 0 891 719
- WO-A-94/01006
- GB-A- 2 323 531

## Beschreibung

Die Erfindung betrifft eine thymuswachstumsstimulierende Säuglingsnahrung und Proteinkomponente und die Verwendung von Arginin und Zink zur Herstellung dieser Proteinkomponente.

Bei der Geburt ist das menschliche Immunsystem noch relativ unreif. Der Thymus spielt dabei vor allem für die Reifung der T-Zellen eine sehr wichtige Rolle. Unreife T-Zellen aus dem Knochenmark werden im Thymus zu immunkompetenten T-Zellen ausdifferenziert. Der Thymus ist besonders in den ersten Lebensmonaten aktiv, später wird das Organ zurückgebildet.

Die Regulation der Thymusfunktion erfolgt durch eine sehr komplexe und sehr speziesspezifische hormonelle Regulation. Dabei sind noch viele Fragen ungeklärt.

In neueren Untersuchungen konnte gezeigt werden, daß die Thymusgröße beim menschlichen Säugling auch von der Nahrung abhängt. Kinder, die Muttermilch erhalten, haben einen signifikant größeren Thymus als Kinder, die eine Formelnahrung erhalten. Außerdem ist bekannt, daß mit Muttermilch ernährte Kinder auf Impfungen im ersten Lebensjahr mit einer stärkeren Antikörperproduktion reagieren als dies Kinder mit Formelnahrung tun. Die Ursache für diese Unterschiede ist aus den bisherigen Untersuchungen am Menschen nicht abzuleiten.

Aus der WO 94/01006 A ist eine Zusammensetzung bekannt, die neben verschiedenen freien Aminosäuren, zu denen auch Arginin gehört, Blütenpollen und weitere Bestandteile enthält, beispielsweise verschiedene Vitamine, verschiedene Metalle und Ribonucleinsäure. Diese bekannte Zusammensetzung dient zur Kontrolle von Rheuma und Arthritis.

Aufgabe der vorliegenden Erfindung ist es, Wege aufzuzeigen, mit denen das Thymuswachstum angeregt wird, so daß es in etwa demjenigen bei der Ernährung mit Muttermilch entspricht.

Gelöst wird diese Aufgabe durch die Lehre der Ansprüche.

Überraschenderweise wurde nämlich festgestellt, daß die Verwendung von Arginin-reichen Säuglingsnahrungen bei gleichzeitiger Supplementierung mit Zink dazu beiträgt, das Thymuswachstum und die Thymusfunktion während der Säuglingsperiode zu stimulieren. Dies führt z.B. zur Erhöhung des Thymusgewichtes bei formelernährten Säuglingen, wodurch der Unterschied zu gestillten Säuglingen reduziert oder sogar ausgeglichen werden kann.

Um die gewünschte Wirkung zu erzielen, müssen somit sowohl Arginin als auch Zink dem kindlichen Organismus in ausreichender Menge zugeführt werden.

Um dieses Ziel zu erreichen, kann man Arginin und Zink zu üblichen Säuglingsnahrungen hinzufügen. Die in der Säuglingsnahrung enthaltene Gesamtmenge an Arginin muß dabei mindestens 3,7 g Arginin/100 g Aminosäuren betragen. Gleichzeitig muß soviel Zink zugesetzt werden, daß ein Zinkgehalt von mindestens 40 mg pro 100 g der insgesamt vorhandenen Aminosäuren erreicht wird. Im Falle von bovinen Milchproteinen sollte dazu die supplementierte Menge Arginin mindestens 0,5 g / 100 g Nahrungsprotein betragen.

Der gewünschte Arginin- und Zinkgehalt in der Säuglingsnahrung kann durch Zugabe besonders argininreicher Proteine oder Peptide, bzw. durch Zugabe von nicht an Proteine und Peptide gebundenem Arginin zusammen mit Zinkverbindungen wie Zinksalzen, oder durch Arginin-Zinkkomplexe, erreicht werden. Zu diesen argininreichen Proteinen und Peptiden zählen z.B Leguminosenproteine, wie Sojaproteine und Erbsenproteine, und die Peptide davon. Zu den Zinkverbindungen zählen Zinkacetat, -gluconat, -chlorid, -lactat, -sulfat, -citrat und -oxid. Bei dem nicht an Proteine oder Peptide gebundenen Arginin handelt es sich dabei zweckmäßigerweise um die L-Form.

Der Kern der vorliegenden Erfindung besteht somit darin, daß man einem Säugling zum Erreichen eines normalen Thymuswachstums eine arginin- und zinkangereicherte bzw. arginin- und zinkreiche Nahrung verabreicht.

Zweckmäßigerweise wird dazu Arginin und Zink einer Säuglingsnahrung und insbesondere der Proteinkomponente dieser Säuglingsnahrung einverleibt.

Allerdings ist es auch möglich, argininreiche Proteine oder Peptide bzw. nicht an Proteine und Peptide gebundenes Arginin zusammen mit dem Zink zusätzlich zu einer üblichen Säuglingsnahrung bzw. Formelnahrung zu verabreichen. So kann beispielsweise freies Arginin zusammen mit Zink in geeigneter Form dem Säuglingskörper einverleibt werden, beispielsweise indem dieser Zusatz aus Arginin und Zink einer üblichen Säuglingsnahrung bei deren Zubereitung einverleibt wird oder indem dieser Zusatz dem Kind getrennt von der Säuglingsmilchnahrung, jedoch in einem kurzen zeitlichen Abstand davon verabreicht wird. Es ist jedoch bevorzugt, Arginin und Zink in die zu verfütternde Säuglingsnahrung von vorne herein einzuverleiben.

Die erfindungsgemäße Säuglingsnahrung bzw. Formelnahrung enthält somit neben einer üblichen Fettkomponente und einer üblichen Kohlenhydratkomponente auch eine Proteinkomponente mit einem Arginingehalt von mindestens 3,7 g pro 100 g der insgesamt vorhandenen Aminosäuren. Als Proteinkomponente können dabei übliche intakte Proteine, hydrolysierte Proteine, Peptide oder deren Bestandteile oder eine oder mehrere nicht an Peptide oder Proteine gebundene L-Aminosäure(n) eingesetzt werden. Zweckmäßigerweise stellt die Proteinkomponente eine Mischung einer oder mehrerer dieser Bestandteile dar.

Für die Herstellung der Proteinkomponente können somit alle üblichen Proteine und deren Bausteine oder Fraktionen, die schon bisher zur Herstellung von Formelnahrungen und Säuglingsnahrungen eingesetzt werden, Anwendung finden. Die Art der Ausgangsbestandteile ist dabei nicht entscheidend, vielmehr kommt es auf den Gehalt an Arginin an.

Derartige Proteine und Peptide können auch mit einer oder mehreren nicht an Peptide oder Proteine gebundene Aminosäure(n) vermengt sein. Zu diesen Aminosäuren zählen nicht nur die freien Aminosäuren sondern auch deren Salze, Ester und weitere übliche Derivate.

Ein Anwendungsgebiet der Erfindung betrifft beispielsweise Säuglingsnahrungen auf Kuhmilchbasis. Übliche Säuglingsnahrungen dieser Art enthalten ausschließlich aus der Kuhmilch stammende Proteine, Peptide bzw. deren Hydrolysate. Um den gewünschten Arginingehalt zu erreichen, kann man die genannten argininreichen Proteine, Peptide sowie deren Hydrolysate hinzugeben, beispielsweise Sojaproteine und -peptide. Zusätzlich oder statt dessen kann man nicht an Proteine oder Peptide gebundenes Arginin zusetzen. Den gewünschten Zinkgehalt erreicht man durch Zugabe der genannten Zinkverbindungen.

Unter einer Formelnahrung wird im Rahmen der vorliegenden Unterlagen eine künstlich hergestellte Säuglingsnahrung bzw. Babynahrung einschließlich Säuglingsmilchnahrung etc. verstanden, die unter Verwendung von tierischen und/oder pflanzlichen einschließlich gegebenenfalls microbiellen Ausgangsstoffen hergestellt wurden, wobei diese Ausgangsstoffe jedoch nicht humanen Ursprungs sind. Es können somit alle für die Herstellung von derartigen künstlichen Formelnahrungen bekannten und/oder geeigneten Ausgangsstoffe eingesetzt werden. Entscheidend ist lediglich der Arginin- und Zinkgehalt.

Bekanntlich enthalten 100 g Protein bzw. Peptid mehr als 100 g Aminosäuren, da bei der Aufspaltung der das Peptid bzw. Protein bildenden Aminosäuresequenz Wasser eingelagert wird, so daß die Summe der aus einem Protein bzw. Peptid entstandenen Aminosäuren größer als 100 g ist.

Um diesem Umstand Rechnung zu tragen, beziehen sich die Grammangaben bezüglich der Argininmenge und auch bezüglich der insgesamt vorhandenen Aminosäuren auf das in Gramm ausgedrückte Molekulargewicht der freien Aminosäuren abzüglich des Molekulargewichtes von Wasser. Dies gilt somit unabhängig davon, in welcher Form die jeweiligen Aminosäuren gebunden sind (z.B. Peptid, Protein) oder nichtgebunden sind (z.B. freie Aminosäuren, Salze, Ester und andere übliche Derivate). Die Berechnung erfolgt somit auf Basis der um den Wasseranteil reduzierten Molekulargewichte der jeweiligen Aminosäuren. Gleiches gilt für die Angabe, daß erfindungsgemäß mindestens 40 mg Zink pro 100 g der insgesamt vorhandenen Aminosäuren eingesetzt wird. Auch in diesem Falle gehen die Molekulargewichte der Aminosäuren ohne den Wasseranteil in die Berechnungen ein. Die angegebene Zinkmenge ist dabei als elementares Zink angegeben, unabhängig davon, in welcher Form das Zink (beispielsweise als Zinksalz) eingesetzt wird.

Wenn im Rahmen der vorliegenden Unterlagen davon die Rede ist, daß die Gesamtmenge an Arginin mindestens 3,7 g pro 100 g der insgesamt vorhandenen Aminosäuren beträgt, dann sind damit auch alle Werte höher als 3,7 g offenbart. So kann erfindungsgemäß beispielsweise eine Arginingesamtmenge von 3,8 g, 3,9 g, 4,0 g, 4,1 g, 4,2 g, 4,3 g, 4,4 g, 4,5 g, 4,6 g usw. zur Anwendung gebracht werden. Gleiches gilt für die Menge an Zink, die mindestens 40 mg pro 100 g der insgesamt vorhandenen Aminosäuren beträgt. Durch diese Angabe sind zumindest alle höheren ganzzahligen Werte für die Mindestgesamtmenge an Zink offenbart, beispielsweise 41, 42. 43, 44, 45, 46, 47, 48, 49 und 50 mg usw. Zweckmäßigerweise beträgt der Zinkgehalt 40-67 mg.

Nach einer bevorzugten Ausführungsform liegen 10 bis 80 Gew.-% des insgesamt vorhandenen Arginins als nicht an Proteine oder Peptide gebundenes Arginin vor. Es kann sich dabei um, wie bereits oben dargelegt, die freie Aminosäure Arginin oder um einfache Salze und Derivate bzw. Mischungen davon handeln.

Durch den Ausdruck 10 bis 80 Gew.-% sind alle dazwischenliegenden Werte und insbesondere alle ganzzahligen Werte und auch alle in den Bereich von 10 bis 80 Gew.-% fallenden kleineren Bereichswerte offenbart. So können beispielsweise 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40 usw. bis 80 Gew.-% bzw. 10 bis 79, 78, 77, 76, 75, 74, 73, 72, 71, 70,69, 68, 67, 66, 65, 64, 63, 62, 61, 60 usw. Gew.-% des insgesamt vorhandenen Arginins als nicht an Proteine oder Peptide gebundenes Arginin vorliegen.

Ferner ist es bevorzugt, als Proteine und Peptide insbesondere solche einzusetzen, die argininreich sind.

Das Zink wird erfindungsgemäß vorzugsweise in Form von Zinksalzen und/oder Arginin-Zink-Komplexen eingesetzt.

Somit können alle für Säuglingsnahrungen bzw. Säuglingsmilchen geeigneten Proteinrohstoffe mit Arginin und/oder argininreichen Proteinen und argininreichen Peptiden sowie mit Zink supplementiert und für die Herstellung von Formelnahrungen verwendet werden.

Die Erfindung wird im folgenden anhand von Beispielen näher erläutert, welche verschiedene Proteinkomponenten bzw. Proteinmischungen beschreiben. Diese Proteinmischungen können durch einfaches Vermengen der dort aufgeführten Bestandteile erhalten werden. Aus diesen Proteinmischungen kann man dann auf per se bekannte Weise Säuglingsnahrungen bzw. Formelnahrungen herstellen, indem man die Proteinmischung mit der erforderlichen Fettkomponente und Kohlenhydratkomponente auf per se bekannte Weise vermischt. Des weiteren können auch übliche und geeignete Zusätze den Säuglingsnahrungen einverleibt werden. Dazu zählen beispielsweise Vitamine, Spurenelemente usw.

Die in den folgenden Beispielen aufgeführten argininangereicherten Proteinmischungen dienen als Basis zur Herstellung von Säuglingsnahrungen die geeignet sind, das Thymuswachstum zu stimulieren.

### Beispiel 1: Proteinmischung auf der Basis von entmineralisiertem Sauermolkenpulver und Na-Caseinat. Das Molkenprotein-Casein-Verhältnis beträgt 60:40

| | |
|---|---|
| Entminineralisiertes Sauermolkenpulver (13,5 % Protein) | 90,9 kg |
| Na-Caseinat (89.9 % Protein) | 9,1kg |
| L-Arginin | 0,4 kg |
| Zinksulfat-Monohydrat | 29,9 g |

Die Aminosäurenzusammensetzung dieser argininangereicherten Proteinmischung ist folgender Tabelle zu entnehmen

| | g/100 g Aminosäuren |
|---|---|
| Asp | 8,7-9,6 |
| Thr | 4,4-4,9 |
| Ser | 4,5-5,5 |
| Glu | 17,5-19,3 |
| Pro | 6,8-7,6 |
| Gly | 1,6-2,0 |
| Ala | 3,5-4,2 |
| Cys | 1,7-2,1 |
| Val | 4,6-5,6 |
| Met | 2,2-2,7 |
| Ile | 4,3-5,3 |
| Leu | 9,6-10,6 |
| Tyr | 2,9-3,6 |
| Phe | 3,7-4,5 |
| His | 2,4-2,9 |
| Lys | 8,5-9,4 |
| Arg | 4,5-5,5 |
| Trp | 1,4-1,7 |

### Beispiel 2: Proteinmischung auf der Basis von Süßmolkenproteinkonzentrat und Na-Caseinat. Das Molkenprotein-Casein-Verhältnis beträgt 50:50

| | |
|---|---|
| Süßmolkenproteinkonzentrat (79,8 % Protein) | 53,5 kg |
| K-Caseinat (91,6 % Protein) | 46,5 kg |
| L-Arginin | 2,9 kg |
| Zinksulfat-Monohydrat | 126,1 g |

Die Aminosäurenzusammensetzung dieser argininangereicherten Proteinmischung ist folgender Tabelle zu entnehmen

| | g/100 g Aminosäuren |
|---|---|
| Asp | 7,8-8,6 |
| Thr | 5,1-5,6 |
| Ser | 4,7-5,7 |
| Glu | 17,7-20,5 |
| Pro | 7,5-8,2 |
| Gly | 1,4-1,8 |
| Ala | 3,1-3,8 |
| Cys | 1,7-2,1 |
| Val | 4,8-5,8 |
| Met | 2,3-2,8 |
| lle | 4,5-5,5 |
| Leu | 8,8-9,7 |
| Tyr | 3,7-4,4 |
| Phe | 3,6-4,3 |
| His | 2,1-2,5 |
| Lys | 7,8-8,7 |
| Arg | 5,6-6,2 |
| Trp | 1,4-1,7 |

## Patentansprüche

1. Thymuswachstumsstimulierende Säuglingsnahrung im wesentlichen aufgebaut aus einer üblichen Fett- sowie Kohlenhydratkomponente und einer Proteinkomponente, wobei die Proteinkomponente aus intakten Proteinen, hydrolysierten Proteinen, Peptiden oder deren Bestandteilen tierischen oder pflanzlichen oder tierischen und pflanzlichen, jedoch nicht humanen Ursprungs oder aus nicht an Peptide oder Proteine gebundenen Aminosäuren oder aus einer Mischung davon aufgebaut ist,
**dadurch gekennzeichnet,**
**dass** die Gesamtmenge an Arginin mindestens 3,7 g pro 100 g der insgesamt vorhandenen Aminosäuren beträgt und
**dass** sie Zink (berechnet als elementares Zink) in einer Menge von mindestens 40 mg pro 100 g der insgesamt vorhandenen Aminosäuren enthält.

2. Säuglingsnahrung nach Anspruch 1,
**dadurch gekennzeichnet,**
**dass** 10 bis 80 Gew.-% des insgesamt vorhandenen Arginins als nicht an Proteine oder Peptide gebundenes Arginin vorliegt.

3. Säuglingsnahrung nach Anspruch 1 oder 2,
**dadurch gekennzeichnet,**
**dass** sie argininreiche Proteine, argininreiche Proteinhydrolysate, argininreiche Peptide oder argininreiche Peptidhydrolysate von Leguminosen, Soja und Erbsen oder Mischungen davon enthält.

4. Säuglingsnahrung nach einem der vorhergehenden Ansprüche,
**dadurch gekennzeichnet,**
**dass** sie das Zink in Form eines oder mehrerer Zinksalze oder als Arginin-Zinkkomplex oder als Mischung davon enthält.

5. Säuglingsnahrung nach einem der vorhergehenden Ansprüche,
**dadurch gekennzeichnet,**
**dass** die Proteinkomponente aus a) Proteinen, hydrolysierten Proteinen, Peptiden und/oder deren Bestandteilen, die aus der Kuhmilch stammen, und b) entweder Sojaproteinen, Sojapeptiden und/oder deren Bestandteilen und/oder c) nicht an Proteine oder Peptide gebundenes Arginin aufgebaut ist.

6. Säuglingsnahrung nach einem der vorhergehenden Ansprüche,
**dadurch gekennzeichnet,**
**dass** sie weitere, für Säuglingsnahrungen übliche und geeignete Zusätze enthält.

7. Proteinkomponente zur Herstellung von thymuswachstumsstimulierenden Säuglingsnahrungen, die aus a) Proteinen, hydrolysierten Proteinen, Peptiden und/oder deren Bestandteilen, die aus der Kuhmilch stammen, und b) entweder Sojaproteinen, Sojapeptiden und/oder deren Bestandteilen und/oder c) nicht an Proteine oder Peptide gebundenes Arginin aufgebaut ist,
**dadurch gekennzeichnet,**
**dass** die Gesamtmenge an Arginin mindestens 3,7 g pro 100 g der insgesamt vorhandenen Aminosäuren ausmacht und
**daß** sie Zink (berechnet als elementares Zink) in einer Menge von mindestens 40 mg pro 100 g der insgesamt vorhandenen Aminosäuren enthält.

8. Proteinkomponente nach Anspruch 7,
**dadurch gekennzeichnet,**
**dass** sie Proteine, Peptide, Arginin und Zink gemäß mindestens einem der Ansprüche 2 oder 4 enthält.

9. Verwendung von Zink zusammen mit Arginin zur Herstellung eines thymuswachstumsstimulierenden Zusatzes für eine Säuglingsnahrung.

10. Verwendung von Arginin zusammen mit Zink zur Herstellung einer thymuswachstumsstimulierenden Proteinkomponente aus intakten Proteinen, hydrolysierten Proteinen, Peptiden oder deren Bestandteilen tierischen oder pflanzlichen oder pflanzlich und tierischen, jedoch nicht humanen, Ursprungs oder aus nicht an Peptide oder Proteine gebundenen Aminosäuren oder aus einer Mischung davon, wobei die Gesamtmenge an Arginin mindestens 3,7 g pro 100 g der insgesamt vorhandenen Aminosäuren ausmacht und wobei das Zink (berechnet als elementares Zink) in einer Menge von mindestens 40 mg pro 100 g der insgesamt vorhandenen Aminosäuren eingesetzt wird.

11. Verwendung nach Anspruch 10, wobei die thymuswachstumsstimulierende Proteinkomponente einer Säuglingsnahrung einverleibt ist.

12. Verwendung nach einem der Ansprüche 9 oder 11, wobei Proteine, Peptide, Arginin und Zink gemäß mindestens einem der Ansprüche 2 bis 5 eingesetzt werden.

## Claims

1. Thymus growth stimulating baby food substantially composed of a common fat component, as well as a common carbohydrate component and a protein component, the protein component being composed of intact proteins, hydrolyzed proteins, peptides or the constituents thereof, of animal or vegetable or animal and vegetable but not human origin, or of amino acids not bound to peptides or proteins or a mixture thereof
**characterized in that**
the total amount of arginine is at least 3.7 g per 100 g of the amino acids present *in toto,* and
that it contains zinc (calculated as elementary zinc) in an amount of at least 40 mg per 100 g of the amino acids present *in toto.*

2. Baby food according to claim 1,
**characterized in that**
10 to 80 wt-% of the arginine present *in toto* are present as arginine not bound to proteins or peptides.

3. Baby food according to claim 1 or 2,
**characterized in that**
it contains arginine-rich proteins, arginine-rich protein hydrolysates, arginine-rich peptides or arginine-rich peptide hydrolysates of leguminoses, soy and peas or mixtures thereof.

4. Baby food according to any one of the preceding claims,
**characterized in that**
it contains zinc in the form of one or more zinc salts or as an arginine-zinc complex or as a mixture thereof.

5. Baby food according to any one of the preceding claims,
**characterized in that**
the protein component is composed of
a) proteins, hydrolyzed proteins, peptides and/or the constituents thereof, which originate from cow milk, and
b) either soy proteins, soy peptides and/or the constituents and/or
c) arginine, which is not bound to proteins or peptides.

6. Baby food according to any one of the preceding claims,
**characterized in that**
it contains further additives usual and suitable for baby foods.

7. Protein component for the preparation of thymus growth stimulating baby foods, which is composed of
a) proteins, hydrolyzed proteins, peptides and/or the constituents thereof, which originate from cow milk, and
b) either soy proteins, soy peptides and/or the constituents thereof and/or
c) arginine, which is not bound to proteins or peptides,
**characterized in that**
the total amount of arginine is at least 3.7 g per 100 g of the amino acids present *in toto,* and
that it contains zinc (calculated as elementary zinc) in an amount of at least 40 mg per 100 g of the amino acids present *in toto*.

8. Protein component according to at least one of the claims 2 to 4,
**characterized in that**
it contains proteins, peptides, arginine and zinc according to at least one of the claims 2 or 4.

9. Use of arginine in conjunction with zinc for preparing a thymus growth stimulating supplement for a baby food.

10. Use of arginine in conjunction with zinc for preparing a thymus growth stimulating protein component composed of intact proteins, hydrolyzed proteins, peptides or the constituents thereof of animal or vegetable or animal and vegetable but not human origin or of amino acids not bound to proteins or peptides or of a mixture thereof, whereby the total amount of arginine accounts for at least 3.7 g per 100 g of the amino acids present *in toto,* and whereby zinc (calculated as elementary zinc) is used in an amount of at least 40 mg per 100 g of the amino acids present *in toto.*

11. Use according to claim 10,
whereby the thymus growth stimulating protein component is incorporated in a baby food.

12. Use according to one of the claims 9 or 11,
whereby proteins, peptides, arginine and zinc are used according to at least one of the claims 2 to 5.

## Revendications

1. Aliment pour nourrissons stimulant la croissance du thymus, principalement composé d'un composant lipidique et glucidique habituel et d'un composant protéinique, le composant protéinique étant composé de protéines intactes, de protéines hydrolysées, de peptides ou de leurs composants d'origine animale ou végétale ou animale et végétale, mais pas d'origine humaine, ou d'acides aminés non liés à des peptides ou à des protéines, ou d'un mélange de ceux-ci,
**caractérisé**
**en ce que** la quantité totale d'arginine est d'au moins 3,7 g pour 100 g de la totalité des acides aminés présents et qu'il contient du zinc (exprimé en zinc élémentaire) à raison d'au moins 40 mg pour 100 g de la totalité des acides aminés présents.

2. Aliment pour nourrissons selon la revendication 1,
**caractérisé**
**en ce que** 10% à 80% en poids de la totalité de l'arginine présente n'est pas sous forme d'arginine liée à des protéines ou à des peptides.

3. Aliment pour nourrissons selon la revendication 1 ou 2,
**caractérisé**
**en ce qu'**il contient des protéines riches en arginine, des hydrolysats de protéines riches en arginine, des peptides riches en arginine ou des hydrolysats de peptides riches en arginines de légumineuses, de soja et de pois, ou des mélanges de ceux-ci.

4. Aliment pour nourrissons selon l'une quelconque des revendications précédentes,
**caractérisé**
**en ce qu'**il contient du zinc sous la forme d'un ou de plusieurs sels de zinc ou de complexe arginine-zinc ou de mélange de ceux-ci.

5. Aliment pour nourrissons selon l'une quelconque des revendications précédentes,
**caractérisé**
**en ce que** le composant protéinique est composé a) de protéines, de protéines hydrolysées, de peptides et/ou de leurs composants qui proviennent du lait de vache et b) de protéines de soja, de peptides de soja et/ou de leurs composants et/ou c) d'arginine non liée à des protéines ou à des peptides.

6. Aliment pour nourrissons selon l'une quelconque des revendications précédentes,
**caractérisé**
**en ce qu'**il contient d'autres additifs habituels et appropriés pour les aliments pour nourrissons.

7. Composant protéinique pour la préparation d'aliments pour nourrissons stimulant la croissance du thymus qui est composé a) de protéines, de protéines hydrolysées, de peptides et/ou de leurs composants, qui proviennent du lait de vache, et b) de protéines de soja, de peptides de soja et/ou de leurs composants et/ou c) d'arginine non liée à des protéines ou à des peptides, **caractérisé**
**en ce que** la quantité totale d'arginine est d'au moins 3,7 g pour 100 g de la totalité des acides aminés présents et qu'il contient du zinc (exprimé en zinc élémentaire) à raison d'au moins 40 mg pour 100 g de la totalité des acides aminés présents.

8. Composant protéinique selon la revendication 7, **caractérisé**
**en ce qu'**il contient des protéines, des peptides, de l'arginine et du zinc selon au moins l'une quelconque des revendications 2 ou 4.

9. Utilisation de zinc conjointement avec de l'arginine pour la préparation d'un additif stimulant la croissance du thymus pour un aliment pour nourrissons.

10. Utilisation d'arginine conjointement avec du zinc pour la préparation d'un composant protéinique stimulant la croissance du thymus composé de protéines intactes, de protéines hydrolysées, de peptides ou de leurs composants d'origine animale ou végétale ou végétale et animale, mais pas d'origine humaine, ou d'acides aminés non liés à des peptides ou à des protéines ou d'un mélange de ceux-ci, la quantité totale d'arginine étant d'au moins 3,7 g pour 100 g de la totalité des acides aminés présents et le zinc (exprimé en zinc élémentaire) étant mis en oeuvre à raison d'au moins 40 mg pour 100 g de la totalité des acides aminés présents.

11. Utilisation selon la revendication 10, le composant protéinique stimulant la croissance du thymus étant incorporé dans un aliment pour nourrissons.

12. Utilisation selon l'une quelconque des revendications 9 ou 11, les protéines, les peptides, l'arginine et le zinc étant mis en oeuvre selon au moins l'une quelconque des revendications 2 à 5.
